Europäisches Patentamt

European Patent Office

Office européen des brevets

(11) Publication number: **0 351 314 B1**

# EUROPEAN PATENT SPECIFICATION

(45) Date of publication of patent specification: **01.02.95**    (51) Int. Cl.⁶: **A61L 33/00**

(21) Application number: **89401997.5**

(22) Date of filing: **11.07.89**

The file contains technical information submitted after the application was filed and not included in this specification

(54) **Medical material and medical implement.**

(30) Priority: **11.07.88 JP 172360/88**
**11.07.88 JP 172361/88**
**09.08.88 JP 198555/88**

(43) Date of publication of application:
**17.01.90 Bulletin 90/03**

(45) Publication of the grant of the patent:
**01.02.95 Bulletin 95/05**

(84) Designated Contracting States:
**BE DE FR GB IT NL SE**

(56) References cited:
**EP-A- 0 051 354       EP-A- 0 092 928**
**EP-A- 0 124 676       EP-A- 0 152 699**
**EP-A- 0 223 626       EP-A- 0 352 330**
**EP-A- 0 407 580       US-A- 3 616 935**
**US-A- 4 720 512**

(73) Proprietor: **TERUMO KABUSHIKI KAISHA**
**No. 44-1, Hatagaya 2-chome,**
**Shibuya-ku**
**Tokyo 151 (JP)**

(72) Inventor: **Hagiwara, Kazuhiko c/o Terumo K.K.**
**2656-1, Ohbuchi**
**Fuji-shi**
**Shizuoka (JP)**
Inventor: **Kitoh, Hitoshi c/o Terumo K.K.**
**2656-1, Ohbuchi**
**Fuji-shi**
**Shizuoka (JP)**
Inventor: **Oshibe, Yoshihiro**
**2-34, Rokkanyama**
**Taketoyo-cho**
**Chita-gun**
**Aichi (JP)**
Inventor: **Ohmura, Hiroshi**
**5-3-1, Rokkanyama**
**Taketoyo-cho**
**Chita-gun**
**Aichi (JP)**

(74) Representative: **Gillard, Marie-Louise et al**
**Cabinet Beau de Loménie**
**158, rue de l'Université**
**F-75340 Paris Cédex 07 (FR)**

EP 0 351 314 B1

**Description**

BACKGROUND OF THE INVENTION

This invention relates to a medical material exhibiting superior anti-thrombotic properties, and a medical instrument or implement making use of such material.

A variety of materials exhibiting anti-thrombotic properties have so far been devised for utilization in pump oxygenators, catheters or artificial hearts. As an example, a method for fixing or immobilizing heparin on the substrate surface has been devised. For instance, coupling of heparin to a substrate through a coupling agent such as a polymeric amine, a dialdehyde or chitosan is disclosed in EP-A- 0 152 699, EP-A- 0 092 928 and EP-A- 0 051 354, respectively. Thus there are known methods consisting in ionically bonding heparin to the substrate, and in covalently bonding heparin to the substrate.

However, with ionic bonding, surface activity is insufficient because of possible detachment of heparin on contact with blood or of too strong bonding with sulfuric group which is critical for demonstration of activities proper to heparin. With covalent bonding, surface activity proper to heparin is similarly insufficient because the bonding point of heparin with the substrate is the bonding point with AT-III. Although an attempt has been made to ionically bond heparin to the cationic surface and to cross-link heparin with glutaraldehyde, the desired effect is not sufficiently persistent because the aldehyde groups may react mainly only with primary amino groups, for example, so that there exist only few primary amino groups in heparin and covalent bonding with the substrate cannot occur in the absence of the primary amino groups in the substrate. While a method including coating a prepolymer is frequently adopted for heparin immobilization, this method has a drawback that polymer peeling or cracking may frequently occur in portions where resilient properties are a requirement.

SUMMARY OF THE INVENTION

It is therefore an object of the present invention to overcome the above problems and to provide a medical material having superior anti-thrombotic properties, and a medical implement utilizing such material as well as a method for manufacturing the same.

In view of the fact that, when it is simply tried to immobilize heparin to the substrate surface, sufficient anti-thrombotic properties cannot be produced, or effective positions are used up for this immobilization, the present inventors have conducted eager researches, and have won a success in immobilizing heparin without using the effective positions and in stably immobilizing heparin even to resilient substances to arrive at the present invention.

Thus there are utilized in heparin the N-sulfuric acid positions which are partially desulfated to produce primary amino groups.

According to the first aspect of the present invention, the medical material comprising a polymer substrate and heparin immobilized thereon to impart anti-thrombotic properties to the substrate, is characterized in that

the substrate has functional groups introduced therein by ozonation through which a compound having two or more primary amine groups (A) is coupled, and

the heparin has primary amino groups (B) obtained by partial desulfation of its N-sulfate moieties, said primary amino groups (B) of the heparin derivative being covalently bonded to said primary amino groups (A) with at least one coupling agent having groups reacting with the primary amino groups (A) and (B).

According to the second aspect of the present invention,

the anti-thrombotic material comprises

a substrate coated with a copolymer of hydroxyethyl methacrylate (HEMA) and methyl methacrylate (MMA) formed of separate segments of HEMA and MMA, said HEMA segment comprising epoxy groups with which a compound having two or more primary amino groups (A) is reacted, and

a heparin derivative having primary amino groups (B) obtained by partial desulfation of its N-sulfate moieties, said primary amino groups (B) of the heparin derivative being covalently bonded to the primary amino group (A) of the substrate with at least one coupling agent having groups reacting with the primary amino group (A) and (B).

Preferably, the compound containing hydroxyethyl methacrylate (HEMA) and methyl methacrylate (MMA) has the contents of not less than 30% of hydroxyethyl methacrylate (MMA).

The substrate and heparin are prepared in this manner and heparin is fixed to the substrate surface. The fixing of the compound having the above functional group on the substrate surface with heparin is performed through one or more coupling agents in the form of covalent bonds. The compounds having at

EP 0 351 314 B1

least two aldehyde groups, such as glutaraldehyde, may be employed as at least one of these coupling agents.

The medical materials obtained by the above described methods according to the first and second aspects of the present invention may be utilized as a variety of anti-thrombotic materials, which in turn may be used above all in the medical implements having portions contacting with blood. Examples of the medical materials include hollow fiber, tube or sheet, whilst examples of the medical implements include oxygenators, pump-oxygenating circuits, catheters or artificial hearts.

Porous membranes or films having numerous micropores are preferably employed as the hollow fibers and oxygenators, these micropores being preferably filled with fine particles of, for example, silica, having lesser particle size than the pore diameter.

## BRIEF DESCRIPTION OF THE DRAWINGS

Fig. 1 is a chart showing desulfation of N-sulfuric acid positions of heparin and changes in the anti-FXa and anti-FIIa activities proper to heparin.

Fig. 2 is a chart showing what value, as measured by the ninhydrin method, the amount of the primary amino group has by the desulfation of the N-sulfuric acid position of heparin.

Fig. 3 is a chart showing surface activities of heparin in samples of medical materials obtained by Example 2-4.

Fig. 4 is a chart showing surface heparin activities in samples of medical materials obtained in Example 3-5.

## DETAILED DESCRIPTION OF THE INVENTION

For better understanding of the present invention, the relation between the different aspects of the present invention will be explained. In sum, the present invention includes two aspects I and II, including in turn separate respective Examples.

(A) First Aspect :

A material in which the functional group is introduced on the substrate with the medium of an oxide, and coupled through a compound having two or more primary amine groups (A) and a coupling agent to heparin, application of the material to a medical implement, and the method for producing the material, which are described in claims 1, 3-7, 9-14 and 16-19.

(B) Second Aspect

The material, application to the implement and the method similar to those of the first aspect mentioned above with the exception that the functional group is introduced to the substrate with the medium of a compound mainly containing HEMA and MMA, which are described in claims 2-13 and 15-19.

(A) First of all, the first aspect of the present invention will be explained.

In the medical material of the present invention, heparin having a portion of N-sulfate groups thereof desulfated and converted into primary amines is fixed to a substrate having functional groups, such as, epoxy group and aldehyde group.

The substrate having the above functional group is first explained.

As the substrate, polypropylene, polyurethane or polyvinyl chloride is generally employed, according to the usage and application.

In general, such substrate itself lacks in the functional group. In this case, the functional group is introduced to the substrate.

According to the present invention, ozone oxidation is utilized for introducing functional groups, such as aldehyde, ketone and epoxy groups.

It has thus been found advisal an expedient to treat the substrate with ozone and to immobilize heparin through the use of these highly reactive functional groups. Although it would be possible to fix heparin directly to these functional groups, the problem of steric hindrance is raised. Thus, the method consisting in introducing a spacer into these functional groups and to fix heparin with the aid of this spacer is most useful because of ease of operation and exhibition of surface activity proper to heparin. According to the invention

3

this spacer consists in a compound having two or more primary amine groups (A) and a coupling agent being able to crosslink amine groups (A) and (B).

By the above technique, the functional groups can be introduced directly into a resilient material, such as polyurethane and polyamide/polyether copolymer, so that heparin can be bonded to the polymer easily without peeling on the polymer surface.

Heparin to be fixed to the substrate having the primary amino group as mentioned above will be explained.

Heparin is well known as a compound exhibiting anti-thrombotic properties, and has a N-sulfuric acid portion in the form of $NHSO_3Na$. Heparin when fixed directly to the substrate surface raises problems, as mentioned above. It is therefore necessary in accordance with the present invention to desulfate and convert a portion of the N-sulfuric acid position into a primary amine. The degree of amination is preferably such that the primary amino groups account for not less than 5% and not more than 15% based on the total number of the amino groups present in heparin, since the amount of the primary amine lesser than the above lower limit lowers the amount of covalent bonds and the amount of the primary amino group more than the upper limit lowers the activity proper to heparin. The preparation of the partially desulfated heparin will be explained in the Examples.

Then, the fixing will be described between the substrate thus obtained including the primary amino group and heparin partially desulfated and primary aminated.

For fixing the substrate and heparin to each other, a compound having at least two aldehyde groups may be used as at least one of the coupling agents, and the primary amine is reacted with the aldehyde group. Such aldehyde compound may include glutaraldehyde. The coupling agent may be exemplified by the aldehyde compound and an epoxy compound, such as polyethylene glycidyl ether. In these cases, it is necessary for a coupling agent having two or more primary amino groups (A), such as polyethylene glycol diamine and polyethylene imine, to be previously coupled to the functional group obtained by an ozonating treatment.

The medical material in which heparin having its N-sulfuric acid partially desulfated to primary amine is fixed to the substrate having the above functional groups represents an anti-thrombotic material which makes use of the anti-thrombotic properties of heparin, and may be employed in a variety of medical implements, such as, for example, oxygenators, pump-oxygenating circuits, catheters or artificial hearts, including portions contacting with the blood. Above all, a pump-oxygenator or a self-retaining catheter formed of the above material is excellent in anti-thrombotic properties, while being stable against breakage, bending or clamping. On the other hand, when the porous membrane having numerous micropores for use as the gas exchange film or membrane, such as hollow fiber, is processed as described above, the hollow fiber exhibiting anti-thrombotic properties may be produced and, when the porous membrane, such as the hollow fiber, is used in the oxygenator, an oxygenator having superior anti-thrombotic properties may be produced.

The micropores of the porous membrane employed in the oxygenator are preferably packed with fine particles lesser in diameter than the pores. The reason is that, when the gas exchange film is porous and hydrophobic and also the functional group are not produced on ozonating treatment, a polymer producing the functional groups need be applied previously, in which case, however, the gas exchange film cannot be coated uniformly with a polymer, so that the anti-thrombotic properties cannot be exhibited fully, while, on the other hand, the membrane is hydrophilized due to fixing of heparin and hence the plasma tends to be exuded and leaked through the pores in case of prolonged circulation.

The packing of the fine particles in the porous film is as described hereafter.

The fine particles may be packed in the porous membrane as described in the Japanese Patent Application KOKAI No. 64374/1987. This will be explained briefly hereinbelow.

A liquid dispersion of fine particles having the particle size lesser than the micropores of the porous membrane is caused to flow through the porous membrane so that the pores are stopped up with the particles.

As the materials for these fine particles, inorganic materials, such as silica, alumina, zirconia, magnesia, barium sulfate, calcium carbonate, silicate, titanium oxide, silicon carbide, carbon black or white carbon, or high polymer latices, such as polystyrene latex, styrene rubber (SBR) latex and nitrile rubber (NBR) latex, may be employed. Silica, above all, is the preferred material. The mean particle size of these fine particles is 0.003 to 1.0 $\mu$m and preferably 0.003 to 0.5 $\mu$m. These fine particles are in the form of a liquid dispersion and applied as such to the gas exchange membrane. A dispersion medium stable against the particles and the gas exchange membrane, such as water and alcohol, may be employed. However, when the dispersion medium is water, and the gas exchange medium is hydrophobic, it is necessary to hydrophilize the surface of the gas exchange membrane by contacting alcohols, such as ethanol and methanol with the surface of the gas exchange membrane prior to causing the liquid dispersion to flow therethrough.

When the gas exchange membrane is a hollow fiber, the liquid dispersion of the fine particles may be passed from the inside of the fiber under a moderate pressure to effect the packing of the fine particles more satisfactorily.

(B) The second aspect of the present invention will now be explained only with reference to the difference thereof from the first aspect.

In the medical material of the present invention, heparin having its N-sulfate groups partially desulfated to primary amine is fixed to the substrate having functional groups.

The substrate having the above functional groups is first explained.

As the substrate, polypropylene, polyvinyl chloride or polyurethane is generally employed, according to the usage and application.

In general, such substrate itself lacks in primary amine. In this case, the primary amine is introduced to the substrate. While there are a variety of methods therefor, the following methods are preferred.

According to the second aspect of the present invention, a polymer including hydroxyethyl methacrylate (HEMA) and methyl methacrylate (MMA) is employed as the material introducing the functional group to the substrate.

This polymer is preferred because the polymer itself has superior adaptability to blood and to living body and high safety, while it can be synthesized and coated relatively easily.

This polymer including HEMA and MMA components is in the form of a block copolymer and the HEMA and MMA components form separate segments A and B, respectively. These segments are separated from each other in the polymer structure. The reason is that, when the respective components are bonded to each other in separate segments, the segments containing MMA having higher water-proofness can be bonded intimately to the substrate. The segments A and B herein mean the fractions or portions containing HEMA and MMA, respectively.

The aforementioned functional groups are caused to exist in the segment A containing hydroxyethyl methacrylate (HEMA). These functional groups may include epoxy group.

For affording these functional groups to the HEMA segment, the compounds containing glycidyl methacrylate (GMA) may be contained in the HEMA segment besides HEMA. Preferably, the epoxy group in GMA and the compound having two or more primary amino groups (A) are reacted with each other to introduce the primary amino groups on the surface.

Similarly, compounds other than MMA may also be contained in the segment B mainly containing MMA.

However it is preferred for the compound containing hydroxyethyl methacrylate (HEMA) and methyl methacrylate (MMA) to contain not less than 30% of hydroxyethyl methacrylate (HEMA). The reason is that, with the lesser amount of HEMA, the surface tends to become hydrophobic and the introduced functional groups tend to appear on the surface only difficultly during the reaction in the aqueous solution.

The fixing of heparin to the substrate is as explained in the first aspect except for those portions relating with ozonating treatment. The application to medical implements, porous membranes used in oxygenators, and the packing of fine particles in the porous membranes are as already described in the first and second aspects.

## EFFECT OF THE INVENTION

According to the present invention, predetermined functional groups are previously introduced onto the substrate, whilst N-sulfuric acid position of heparin is partially desulfated and converted into primary amino groups and the functional groups of heparin and the substrate are bonded to each other directly or with the intermediary of coupling agents for immobilizing heparin to the substrate. Thus the medical materials and, above all, the medical implements making use of these materials, such as the oxygenators, are markedly improved in anti-thrombotic and anti-plasma-leakage properties, with the result that the materials or implements can be used over an extended period of time.

## EXAMPLES

The first aspect of the present invention will be explained with reference to several Examples.

Preparation of Partially N-desulfated Heparin

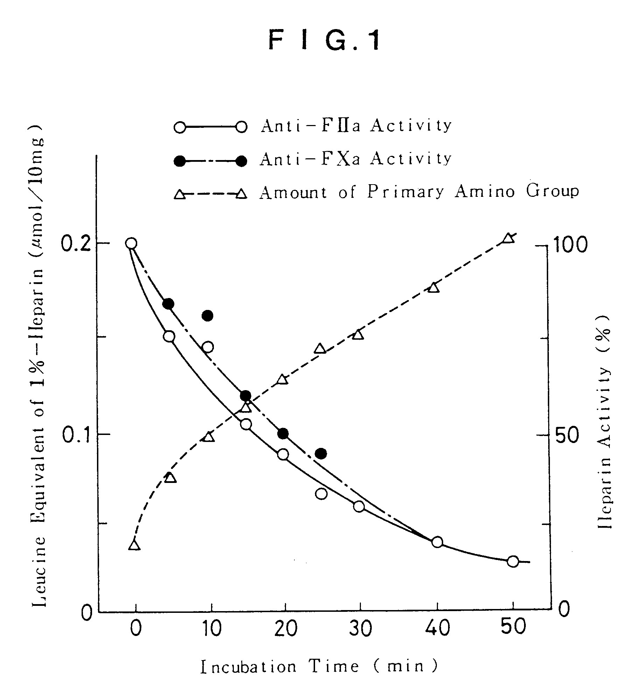

Commercially available heparin was dissolved in distilled water to prepare a 10%-heparin solution. 0.4 ml of 5.5 N $H_2SO_4$ was added to 10 ml of this heparin solution and reacted at 95°C. The reaction mass was sampled periodically and the increase in the amount of the primary amino groups was measured by a ninhydrin method, whilst anti-FXa and anti-FIIa activities, among the heparin activities, were measured by the synthetic substrate method making use of S-2222 and S-2238. The results are shown in Fig.1.

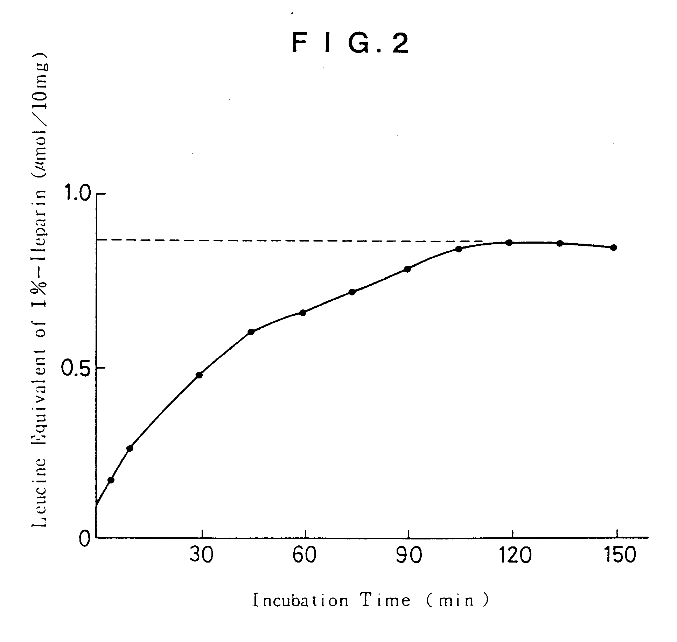

A reaction was conducted under the condition that, a literature say, all the sulfoamino groups in heparin would be desulfated, and the reaction mass was sampled periodically and the increase in the amount of the primary amino groups was measured. The results are shown in Fig. 2.

It is seen from Fig. 2 that, when the sulfoamino groups are converted as the whole into primary amino groups, the amount of the primary amino groups of 0.9 $\mu$mol/10 mg is indicated by the ninhydrin method.

On the other hand, it is seen from Fig. 1 that the heparin activity is lowered with increase in the amount of primary amino groups and the excess desulfation of N-sulfuric acid of heparin is not preferred. For maintaining the heparin activity to some extent, it is preferred to maintain the primary amino groups in heparin at the level of 10 to 20% in -N sulfuric acid from the value of the amino group when converted into the whole sulfoamino group in Fig. 2 and from that of the amino group of Fig. 1. Hence, in the Examples to follow, aminated heparin obtained by the reaction continuing for 15 minutes is employed.

Meanwhile, the ninhydrin reagent is prepared by dissolving 2 g of ninhydrin and 0.3 g of hydrindantin in 75 ml of methyl-cellosolve and adding 25 ml of 4N sodium acetate (pH, 5.5).

To 0.75 ml of a sample to be tested is added 0.5 ml of the ninhydrin reagent and the resulting mixture is heated in boiling water for 15 minutes. The heated mixture was cooled rapidly and admixed with 5 ml of 25%-ethanol and the light absorbance was measured at 570 nm. The quantity of the amino groups was determined by the coloration degree of leucine.

In Fig. 1, the mark ○ indicates anticoagulancy against activated second blood clotting factor, whereas the mark ● indicates anticoagulancy against activated tenth blood clotting factor. There are a clotting factor for which heparin exhibits its anticoagulancy only in the high molecular weight range and a clotting factor for which heparin exhibits its anticoagulancy even in the low molecular weight range. In Fig. 1, activated second and activated tenth factors, representative of the clotting factors, are taken as examples to illustrate that heparin shows about the same anticoagulant activities for both the high and low molecular weight ranges.

It is seen from Figs. 1 and 2 that the amount of the primary amino groups in heparin is increased with incubation time, but the activities proper to heparin are lowered gradually, as shown in Fig. 1. It is therefore necessary to turn the N-sulfuric acid position of heparin into primary amine in the region in which the activities proper to heparin are not lowered inappropriately.

The first aspect of the present invention will be explained with reference to several Examples.

I. Introduction of Functional Groups to the Substrate

Example 1-1

Sheets of PEBA 6333SAOO and 2533SAOO, both a block copolymer of nylon and polyether, produced by Atochem Inc. and NKY-9LH, a solvent-soluble polyurethane, were prepared and termed A, B and C, respectively. The sheet A was processed by an ozonator produced by Nippon Ozone Co., Ltd. under conditions of 0.8 lit/min of $O_2$ and 50°C for 10 minutes, while the sheets B and C were processed for 20 minutes under otherwise the same conditions. These processed sheets were termed A1, B1 and C1, respectively.

The changes in the ratio of the surface element composition were measured by ESCA before and after the ozonating treatment. The results are shown in Table 1-1.

The aldehyde on the suface was ascertained using a Shiff's reagent. It was found that the samples subjected to ozonating treatment A1, B1 and C1 were stained in a red purple to blue purple tint, whereas those not subjected to ozonating treatment A, B and C were not stained.

Table 1-1

| Analysis of Surface Composition by ESCA | | | | | | |
|---|---|---|---|---|---|---|
| | A | A1 | B | B1 | C | C1 |
| O | 17.12 | 29.16 | 21.25 | 25.85 | 18.39 | 22.43 |
| N | 0.80 | 1.43 | 1.70 | 4.78 | 3.36 | 4.17 |
| C | 82.05 | 69.74 | 77.04 | 69.36 | 78.25 | 73.40 |

II. Preparation of Partially N-Desulfated Heparin

Example 1-2

The commercially available heparin was dissolved in distilled water to prepare a 10%-heparin solution. 10 ml of this heparin solution were charged into 0.4 ml of 5.5N sulfuric acid and incubated at 97°C for ten minutes.

The amount of the primary amino groups in the produced heparin was 11% of total amino groups in untreated heparin. 11% were inclusive of those possessed by heparin from the outset and those produced by desulfation of sulfuamino groups.

III. Heparin Fixation to Substrate and Evaluation

Example 1-3

The sheets A, B, C, A1, B1 and C1, produced in accordance with the Example 1-1 were immersed at 45°C for 24 hours in 1.7% PGD-10 (polyethyleneglycol diamine) or 0.5% polyethylene imine (PEI), manufactured by BASF, adjusted to pH of 10. Then, acetic acid buffer solutions, pH 4.5, were prepared of 0.5% partially desulfated heparin reacted according to Example 1-2 and heparin prior to the reaction and the films were immersed in these solutions at 45°C for 24 hours. The films were then immersed in a 2.5% glutar aldehyde acetic acid buffer solution, pH 4.5, at room temperature for 24 hours and then in 1% NaBH$_4$ carbonic acid buffer solution, pH 10, at room temperature for four hours.

The sheets thus processed were immersed in 0.01N hydrochloric acid and stained in toluidin blue. It was found that the sheets A, B and C not subjected to ozonating treatment were scarcely stained.

The sheets subjected to ozone treatment A1, B1 and C1 were stained only slightly when reacted with heparin prior to reaction, while the same sheets when reacted with partially desulfated heparin were stained in purple to red purple color.

The sheets A, B, C, A1, B1 and C1 produced in accordance with Example 1-1 and processed similarly except the process of reacting with PEI and PGD-10 were stained in blue purple color when they were reacted with partially desulfated heparin after ozonating treatment.

Example 1-4 Anti-Thrombotic Properties of Tube on which Heparin was Fixed

A tube having an inside diameter of 1.4 mm was prepared from a polymer B shown in Example 1-1. Also a polyurethane tube having an inside diameter of 1.4 mm and coated on its inner surface with polymer C was prepared. The inner sides of these tubes were ozonated under the same ozonating conditions as those of Example 1-1. Partially desulfated heparin obtained in accordance with Example 1-2 was fixed on these inner sides in accordance with Example 1-3. These samples were termed B2, C2, B3 and C3. The samples without ozonating treatment of the tube and subjected to heparin fixing treatment were also prepared and termed B4, C4, B5 and C5.

In the samples B2, C2, B4 and B4, and samples B3, C3, B5 and C5, polyethylene glycol diamine and polyethylene imine were used respectively as the coupling agents between the substrate and heparin.

These tubes were rinsed with a borate buffer solution, pH 9, for 15 hours and then rinsed with a phosphate buffer solution, pH 7.4, for two hours. Anti-thrombinic surface properties of each tube were then measured.

In more detail, a tube on which heparin was fixed was cut to a length of 56 cm and 0.5 ml of 0 to 10 U/cc of thrombin (4% Alb saline solution) was introduced into the tube and allowed to contact with the inner

EP 0 351 314 B1

surface of the tube by use of a rotary mixer for 15 minutes. The thrombinic concentration of the inside liquid was measured to compute the amount of thrombin adsorbed to the inner tube surface. The tubes to which thrombin was adsorbed was washed with a saline solution. Then, 1.0 ml of a detergent liquid (0.6 mM S-2238) was caused to flow through the inside of the tube at the rate of 2 ml/min and the liquid flowing out of the tube was caused to flow dropwise into 0.2 ml of 50% acetic acid to terminate the reaction. The light absorbancy of the reaction solution was measured and a calibration curve for color developing properties of S-2238 with respect to the amount of thrombin adsorbed to the inner surface was prepared.

Then, 1U/cc of AT-III was introduced into a tube to which 10U/cc of thrombin was adsorbed. After incubation, S-2238 was similarly subjected to color development with thrombin remaining on the inner surface and the amount of thrombin remaining on the inner surface was calculated from the degree of color development and the calibration curve.

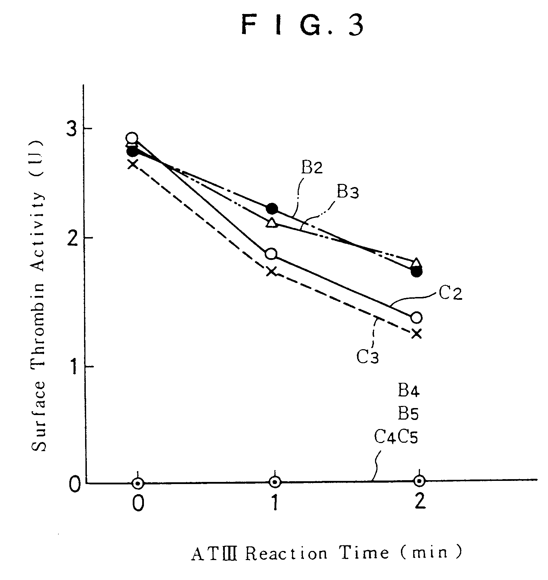

The changes in the amount of thrombin remaining on the inner surface with the incubation time for AT-III being changed are shown in Fig.3. It was found that the samples subjected to ozonating treatment exhibited thrombin activities.

The tube was further rinsed with plasma for 30 minutes and heparin washed out into plasma was washed. The Chandler's loop test was then conducted.

The results are shown in Table 1-2. It is seen from this table that the effect of ozonating treatment and heparin immobolization manifests itself in the actual blood.

The method and the significance of the Chandeller's loop test are explained hereinbelow.

By measurement of the time during which the fresh blood not treated for anticoagulancy becomes coagulated, it is possible to evaluate the level of anti-thrombotic properties of the material contacted by the blood.

This tube was cut to a length of 20 cm and 88 $\mu$l of fresh blood sampled from the veins of the rabbit's ears was intorduced into the tube segment which was bent into a self-closed loop and turned at 8 rpm.

The time until she blood is coagulated and start to turn with the tube was measured.

From the above results, it is seen that heparin is immobilized by ozone processing and anti-thrombotic propeties are afforded to the samples.

8

## Table 1-2 Chandler's Loop Test
### (Total Blood Coagulation Time)

| | B2 | B3 | C2 | C3 | B4 | B5 | C4 | C5 |
|---|---|---|---|---|---|---|---|---|
| RUN 1 | 40'15" | 60'< | - | - | | | - | - |
| RUN 2 | | | 27'25" | 60'< | 10'20" | 12'40" | 11'55" | 12'20" |
| RUN 3 | 60'< | 60'< | | | | | | |
| RUN 4 | | | 26'30" | 30'35" | 12'10" | 13'45" | 11'20" | 10'05" |
| RUN 5 | 52'50" | 60'< | 31'40" | 60'< | | | | |
| RUN 6 | | | | | 9'40" | 10'15" | 10'30" | 11'10" |

Example 1-5

Using a polymer B, the catheter with an inner diameter of 1.4 mm, an outer diameter of 2.2 mm and a length of 500 mm was produced. A polymer C was coated on the inner and outer surfaces of polyurethane to produce similarly the catheter with an inner diameter of 1.4 mm, an outer diameter of 2.2 mm and a length of 500 mm. The inner and outer surfaces of this catheter were treated with ozone and heparin was fixed to the inner and outer surfaces of the catheter similarly to the samples B3 and C3 of Example 1-4 to produce the catheter with heparin fixed thereto (samples B6 and C6). On the other hand, heparin was immobilized to the inner and outer surfaces of the catheter, similarly to the samples B4, C4, B5 and C5, without ozonating treatment, to produce catheters (B7, C7, B8 and C8).

With cannulation being made to left and right femoral veins of a mongrel weighting 15 kg and lactate ringel solution was caused to flow in the inside at a rate of 5 ml/hr, the catheter was left for 8 hours and then taken out to observe visually the degree of formation of thrombi in the catheter.

Thrombi were scarecely formed on the inner and outer sides of the catheters B6 and C6, whereas the inner sides of the Catheters B7, C7, B8 and C8 were substantially occulated while a large number of thrombi were affixed to the outer sides, thus demonstrating the favorable result of heparin immobilization by ozonating treatment.

Example 1-7

Polyurethane soluble in a solvent (NKY-9LH produced by Nippon Polyurethane Co., Ltd.) was coated on a soft vinyl chloride tube (CBT-650 produced by Terumo Kabushiki Kaisha). This tube was treated by an ozone generator for ten minutes at a flow rate of 0.8 lit/min of $O_2$ and at 50°C. This tube was immersed at 45°C for 20 hours in 0.05% polyethylene imine (PEI) and then at 45°C for four hours in 0.5% heparin (pH 4.0, 0.1 M acetic acid buffer).

On the other hand, polyepoxy compound (Denacol EX-421 produced by Nagase Co., Ltd.) and a diepoxy compound (Denacol EX-313 produced by Nagase Kasei Co., Ltd.) were dissolved in 10 mM of an acetic acid buffer, pH 4.0 to produce an aqueous solution of 5% polyepoxy compound and 10% diepoxy compound. Water-insoluble components were precipitated by centrifugation at 3000 rpm for ten minutes. The above tube was immersed in the recovered supernatant liquids as tubes I and II. 10 mM of acetic acid buffer solution not containing epoxy compounds, pH 4.0, was immersed in the tubes processed as above as tube III.

These tubes were reacted at 45°C for 20 hours and 44 hours, each tube showing as I-20 (hr), I-44(hr), II-20 (hr) and II-44 (hr). The tubes were then immersed for 16 hours in 1M ethanolamine, pH 10, before washing with water. These samples were stained with toluidine blue and the results as shown in Table 1-3 were obtained.

Table 1-3

| hr | Ex. I | Ex. II | Comp. Ex. III |
|----|-------|--------|---------------|
| 20 | + | + | - |
| 44 | + + + | + + + | - |

Example 1-8

A sheet of urethane soluble in a solvent (NKY-9LH) was prepared by dipping. This sheet was processed as in Example 1-7 and heparin was immobilized on this sheet. The sheet was then immersed in a polyepoxy and diepoxy compounds same as in Example 1-7 for 20 hours or 44 hours to perform an epoxy treatment (i), followed by ethanol amine treatment (ii) including immersion in 1M ethanol amine solution, pH 10.0, and further immersion treatment (iii) in 1M sodium chloride, pH 10, to remove ion-bonding heparin.

The ratio of the elementary composition of each sheet was measured at each stage of (i) to (iii). The results are shown in Table 1-4.

Table 1-4

| (S element composition ratio in %) | | | | | |
|---|---|---|---|---|---|
| | Ex. 1 | | Ex. 2 | | Cmp. Ex. III | |
| hr | 20 | 44 | 20 | 44 | 20 | 44 |
| (i) | 1.91 | 2.07 | 2.03 | 1.96 | 2.03 | 2.03 |
| (ii) | 0.99 | 1.12 | 0.92 | 1.24 | 0.57 | 0.51 |
| (iii) | 0.93 | 1.17 | 0.94 | 1.28 | 0.60 | 0.53 |

Example 1-9

Polyurethane soluble in a solvent (NKY-9LH) was coated on the inner surface of a soft polyvinyl chloride tube (inside diameter, 1.4 mm) which was then treated in the same way as in Example 1-7. Heparinated tubes were produced in this manner for each of I-44 (hr), II-20 (hr) and II-4 (hr). Evaluation of anti-thrombotic properties was made of each tube by the Chandeller loop method.

For comparison and reference, the sample III in Example 1-7 was employed.

The evaluation was made on these samples directly and after incubation with 6% albumin for 24 hours three times and washing desorbed excess heparin thoroughly. The results are shown in Table 1-5.

It is seen from the above results that anti-thrombotic properties have been realized by fixing heparin to the epoxy compounds. It is felt that excess heparin has been substantially removed by processing with 1M ethanol amine processing at pH 10.

Table 1-5

| | Evaluated Directly | | | | Evaluated After Incubation with 6%-Albumin | | | |
|---|---|---|---|---|---|---|---|---|
| | RUN 1 | RUN 2 | RUN 3 | RUN 4 | RUN 1 | RUN 2 | RUN 3 | RUN 4 |
| I-44 | < 60' | < 60' | < 60' | < 60' | < 60' | < 60' | < 60' | < 60' |
| II-20 | 25'45" | 32'20" | 27'10" | 40'55" | 20'25" | 23'15" | 19'55" | 30'45" |
| II-44 | < 60' | < 60' | < 60' | < 60' | < 60' | < 60' | < 60' | < 60' |
| III | 10'35" | 12'05" | 8'50" | 7'20" | 8'30" | 9'15" | 5'50" | 13'30" |

Example 1-10

Polyurethane soluble in a solvent (NKY-9LH) was applied to the inner and outer surfaces of a soft vinyl chloride tube (inside diameter, 1.4 mm; outside diameter,2 mm) and heparinated tube was produced in the same way as in Example 1-7 in accordance with I-44 (epoxy compound 0% was processed as Comparative

12

Example III for 44 hours).

With cannulation into left and right femoral veins and artery of a mongrel weighing 15 kg, and causing the inner side to flow with a lactate ringel at the rate of 5 ml/hr, the tube was left for 8 hours and then taken out to observe the degree of formation of thrombi visually. The sample I-44 was substantially free from thrombi, whilst in III numerous thrombi were noticed on both the inner and outer sides.

The second aspect of the present invention will be explained with reference to several Examples.

I. Introduction of Functional Groups to Substrate

Example 2-1

Polymers P1 to P4 of various compositions shown in the following Table 2-1 were prepared and coated on flat microporous polypropylene films. The polymer solutions were adjusted by diluting a 15% polymer solution in methyl cellosolve with methanol or a 9 : 1 methanol : acetone solution into a 2.5% polymer solution.

It is noted that epoxy groups are introduced into the segment containing hydroxyethyl methacrylate (HEMA) by bonding glycidyl methacrylate (GMA) whilst acrylic acid (AA) is introduced into the segment containing methyl methacrylate (MMA).

Table 2-1

| Polymer Composition | | |
| --- | --- | --- |
| | HEMA segment HEMA/GMA (wt.%) | MMA segment MMA/AA (wt.%) |
| P1 (comparision) | 80/0 | 20/0 |
| P2 | 68/12 | 17/3 |
| P3 | 55/25 | 17/3 |
| P4 | 40/40 | 17/3 |

II Preparation of Partially N-Desulfated Heparin

Example 2-2

The commercially available heparin was dissolved in distilled water to prepare a 10%-heparin solution. 10 ml of this heparin solution was charged into 0.4 ml of 5.5N sulfuric acid and incubated at 97°C for ten minutes.

The primary amino groups in the total amino groups in the produced heparin accounted for 11% of total amino groups in untreated heparin. 11% were inclusive of those possessed by heparin from the outset and those corresponding to the N-sulfuric acid positions desulfated and turned into primary amines.

III. Fixation of Heparin to Substrate and Evaluation

Example 2-3

A film prepared in accordance with Example 2-1 was immersed in 0.1% ethylene diamine and 1.7% PGD-10 (polyethylene glycoldiamine), adjusted to pH of 10, at 45°C and for 24 hours. Then, acetic acid buffer solution of pH 4.5 was prepared for each of 0.5% partially N-desulfated heparin reacted in accordance with Example 2-2 and heparin prior to reaction and the film was immersed in this solution at 45°C for 24 hours. The film was immersed in a 2.5% glutar aldehyde acetic acid buffer solution of pH 4.5 at room temperature for 24 hours and then in a 1% $NaBH_4$ carbonic acid buffer solution, pH 10, at room temperature for four hours.

These processed films were immersed in 0.01N hydrochloric acid and stained with toluidin blue. The films immersed in heparin not desulfated were substantially not stained, whereas the films immersed in partially desulfated heparin of polymers P2 to P4 were stained in red purple color. However, the polymer P1 was not stained. The films were also immersed in a borate buffer solution of pH 9 for 15 hours to remove ion-bonded heparin and dyed in a similar manner. It was found that there was no change except that the heparin immersed in film could not be stained.

Example 2-4 Ion Bonding Properties between Heparin and Substrate in Neutral Region (comparative example)

The film prepared in accordance with Example 2-1 was immersed in 0.1% ethylene diamine and 1.7% PGD-10 (polyethylene glycol diamine), adjusted to pH of 10, at 45°C for 24 hours. A 0.5% acetic acid buffer solution of pH 4.5 was prepared for each of partially desulfated heparin racted in accordance with Example 2-2 and heparin prior to reaction, and the film was immersed in this solution at 45°C for 24 hours. The film was then immersed in a phosphate buffer solution of pH 7.4 at room temperature for 24 hours and stained in the similar manner and was found to be scarcely stained.

From the fact stated, it follows that heparin and the substrate are not bonded ionically in the neutral range.

Example 2-5 Anti-Thrombotic Properties of Heparinized Tube

The polymer shown in Example 2-1 was coated on polyamide tubes each having the inside diameter of 1.4 mm and partially desulfated heparin produced in Example 2-2 similarly to Example 2-3 was fixed to the polymer. These tubes were washed in a borate buffer solution of pH 9 for 15 hours and then in a phosphate buffer solution of pH 7.4 for two hours and anti-thrombotic surface properties of each tube were measured. In more detail, a tube on which heparin was fixed was cut to a length of 56 cm and 0.5 ml of 0 to 10 U/cc of thrombin (4% Alb saline solution) was introduced into the tube and allowed to contact with the inner surface of the tube by use of a rotary mixer for 15 minutes. The thrombinic concentration of the inside liquid was measured to compute the amount of thrombin adsorbed to the inner tube surface. The tube to which thrombin was adsorbed was washed with a saline solution. Then, 1.0 ml of a detergent liquid (0.6 mM S-2238) was caused to flow through the inside of the tube at the rate of 2 ml/min and the liquid flowing out of the tube was caused to flow dropwise into 0.2 ml of 50% acetic acid to terminate the reaction. The light absorbancy of the reaction solution was measured and an analytical curve for color developing properties of S-2238 with respect to the amount of thrombin adsorbed to the inner surface was prepared.

Then, 1U/cc of AT-III was introduced into a tube to which 10U/cc of thrombin was adsorbed. After incubation, S-2238 was similarly subjected to color development with thrombin remaining on the inner surface and the amount of thrombin remaining on the inner surface was calculated from the degree of color development and the analytical curve.

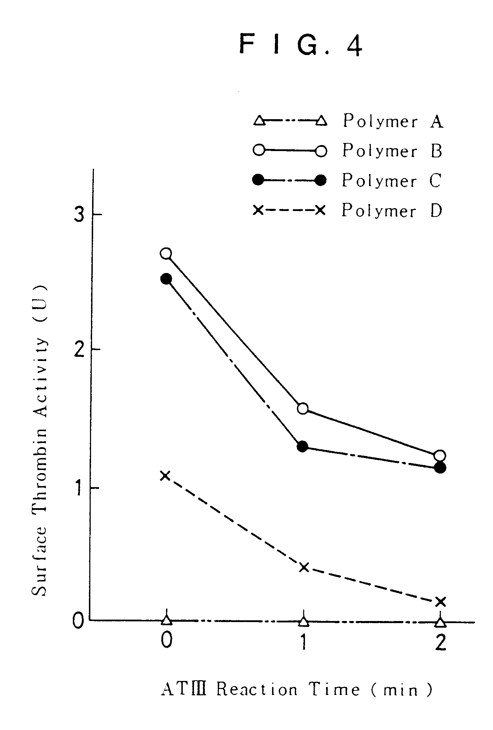

The changes in the amount of thrombin remaining on the inner surface with the incubation time for ATIII being changed are shown in Fig.4. It was found that the samples stained to red to purple color exhibited activities.

It is seen from Fig.4 that the polymer to which glycidyl group was introduced exhibited surface heparin activities and, above all, the polymer having higher HEMA contents exhibit such activities.

IV. Fixation of Heparin to Oxygenator and Evaluation

Example 2-6

A polypropylene hollow fiber type oxygenator having the inside diameter of 200 $\mu$m, wall thickness of 25 $\mu$m, porosity of 4% and a mean pore diameter of 700 Å (film surface, 0.8 m$^2$) was coated with the polymer P2 shown in Table 2-1. This oxygenator was immersed in 1.7% PGD-10 (polyethylene glycol diamine), adjusted to pH 10, at 45°C for 24 hours.

An acetic acid buffer solution, pH 4.5, of 0.5% partially N-desulfated heparin reacted in accordance with Example 3-2 was prepared and the oxygenator was immersed in this solution at 45°C for 24 hours. The oxygenator was immersed in an acetic acid buffer solution of pH 4.5 with 2.5% of glutar aldehyde at room temperature for 24 hours. The oxygenator was then immersed in 1% NaBH$_4$, pH 10 carbonic acid buffer solution at room temperature for four hours to produce an oxygenator A.

On the other hand, ethanol was caused to flow into the similar oxygenator via blood inlet to hydrophilize the gas exchange film. After substitution with distilled water, a dispersion of colloidal silica of mean particle size of 0.0125 $\mu$m in water was caused to flow into the oxygenator and filtrated through the gas exchange film for packing the silica in the pores. The distilled water was then introduced for dispalcing the dispersion in water of silica remaining in the inside of the gas exchange film and the oxygenator was then dried. The oxygenator was processed in the similar manner as for the oxygenator to produce the oxygenator B.

An oxygenator not coated with the polymer shown in Table 2-1 was prepared for comparison and reference as an oxygenator C.

Example 2-7 Evaluation of Anti-Thrombotic Properties

An A-V shunt was performed for femoral veins and artery of a mongrel weighing 25 kg, with respect to oxygenators A, B and C on which heparin was immobilized in accordance with Example 2-6 and the blood was circulated at the maximum rate of 400 ml/min.

There was no lowering of the flow rate caused by the increased pressure loss during circulation for eight hours through the oxygenators A and B.

The flow rate was seen to be lowered after 9 hours in the oxygenator A, whereas there was no lowering of the flow rate even after 12 hours in the oxygenator B.

Conversely, circulation was rendered unfeasible in two hours in the oxygenator C due to formation of thrombi in the oxygenator C. Plasma leakage was seen to occur after 6 hours in the oxygenator A, whereas such plasma leakage was not noticed even after 12 hours in the oxygenator B.

**Claims**

1. A medical material comprising a polymer substrate and heparin immobilized thereon to impart anti-thrombotic properties to the substrate, characterized in that

   the substrate has functional groups introduced therein by ozonation through which a compound having two or more primary amine groups (A) is coupled, and

   the heparin has primary amino groups (B) obtained by partial desulfation of its N-sulfate moieties, said primary amino groups (B) of said heparin derivative being covalently bonded to said primary amino groups (A) with at least one coupling agent having groups reacting with the primary amino groups (A) and (B).

2. A medical material comprising a substrate and heparin immobilized thereon to impart anti-thrombotic properties to the substrate, characterized in that

   the substrate is coated with a copolymer of hydroxyethyl methacrylate (HEMA) and methyl methacrylate (MMA) formed of separate segments of HEMA and MMA, said HEMA segment comprising epoxy groups with which a compound having two or more primary amino groups (A) is reacted, and

   the heparin has primary amino groups (B) obtained by partial desulfation of its N-sulfate moieties, said primary amino groups (B) of said heparin derivative being covalently bonded to said primary amino group (A) with at least one coupling agent having groups reacting with the primary amino group (A) and (B).

3. Material according to one of claims 1 or 2, wherein said desulfated heparin has between 5 and 15% primary amino groups with respect to the sum of the original primary amino groups and the original N-sulfate moieties in the native heparin.

4. Material according to any one of claims 1 to 3, wherein said partial desulfation is carried out in aqueous sulfuric acid.

5. Material according to any one of claims 1 to 4, wherein said substrate is a member selected from the group consisting of polypropylene, polyvinyl chloride and polyurethane.

6. Material according to claim 1, wherein said substrate is a polyamide/polyether copolymer.

7. Material according to any one of claims 1 to 6, wherein said coupling agent is glutaraldehyde.

8. Material according to claim 2 wherein said copolymer containing hydroxyethyl methacrylate (HEMA) and methyl methacrylate (MMA) contains not less than 30% of hydroxyethyl methacrylate (HEMA).

9. A medical implement wherein at least a portion thereof contacting with the blood is formed of the material according to any one of claims 1 to 8.

15

10. A hollow fiber wherein at least a portion thereof contacting with the blood is formed of the material according to any one of claims 1 to 8.

11. An oxygenator comprising a porous membrane having a multiplicity of micropores as a gas exchange membrane, wherein a surface of the membrane on which the blood is circulated is formed of the material according to any one of claims 1 to 8.

12. The oxygenator according to claim 11 wherein a multiplicity of fine particles having a particle size lesser than the diameter of the pore is packed in said micropores of said porous membrane.

13. The oxygenator according to claim 12 wherein said fine particles are silica particles.

14. A method for producing a medical material according to claim 1 which comprises the steps of
    (a) treating a polymer substrate by ozonation to introduce functional groups therein ;
    (b) coupling a compound having two or more primary amino groups to said functional groups, and
    (c) covalently bonding the amino groups of the oxidized substrate and the amino groups of partially desulfated heparin by means of a coupling agent.

15. A method for producing a medical material according to claim 2 which comprises the steps of
    (a) coating a substrate with a copolymer of HEMA and MMA made up of separate segments of HEMA and MMA, the HEMA segment containing a compound having epoxy groups,
    (b) reacting a compound having two or more primary amino groups with said coated substrate, and
    (c) covalently bonding the amino groups of the substrate and the amino groups of partially desulfated heparin by means of a coupling agent.

16. The method according to one of claims 14 or 15 wherein said coupling agent is a compound having at least two aldehyde groups.

17. The method according to claim 16 wherein said compound having at least two aldehyde groups is glutaraldehyde.

18. The method according to any one of claims 15-17, wherein said compound containing epoxy groups is glycidyl methacrylate.

19. A method for producing an oxygenator as defined in claim 11, wherein the material is prepared according to the method of any one of claims 14-18.

**Patentansprüche**

1. Medizinisches Material, umfassend ein Polymersubstrat und darauf immobilisiertes Heparin, um dem Substrat Anti-Thromboseeigenschaften zu verleihen, **dadurch gekennzeichnet**, **daß** das Substrat durch Ozonisierung eingeführte funktionelle Gruppen aufweist, durch die eine Verbindung mit zwei oder mehreren primären Amingruppen (A) gebunden ist, und das Heparin primäre Aminogruppen (B) aufweist, die durch eine partielle Desulfurierung seiner N-Sulfat-Gruppen erhalten wurden, wobei die primären Aminogruppen (B) des Heparinderivats mit mindestens einem Verknüpfungsmittel, das Gruppen aufweist, die mit den primären Aminogruppen (A) und (B) reagieren, kovalent an die primären Aminogruppen (A) gebunden sind.

2. Medizinisches Material, umfassend ein Substrat und darauf immobilisiertes Heparin, um dem Substrat Anti-Thromboseeigenschaften zu verleihen, **dadurch gekennzeichnet**, **daß** das Substrat mit einem Copolymer aus Hydroxyethylmethacrylat (HEMA) und Methylmethacrylat (MMA), das aus getrennten Segmenten des HEMA und MMA gebildet ist, beschichtet ist, wobei das HEMA-Segment Epoxygruppen umfaßt, mit denen eine Verbindung mit zwei oder mehreren primären Aminogruppen (A) umgesetzt wird, und

das Heparin primäre Aminogruppen (B) aufweist, die durch eine partielle Desulfurierung seiner N-Sulfat-Gruppen erhalten wurden, wobei die primären Aminogruppen (B) des Heparinderivats mit mindestens einem Verknüpfungsmittel, das Gruppen aufweist, die mit den primären Aminogruppen (A) und (B) reagieren, kovalent an die primären Aminogruppen (A) gebunden sind.

3. Material nach einem der Ansprüche 1 oder 2, **dadurch gekennzeichnet, daß** das desulfurierte Heparin in Bezug auf die Summe an ursprünglichen primären Aminogruppen und ursprünglichen N-Sulfat-Gruppen in dem natürlichen Heparin zwischen 5 und 15% primäre Aminogruppen aufweist.

4. Material nach einem der Ansprüche 1 bis 3, **dadurch gekennzeichnet, daß** die partielle Desulfurierung in wäßriger Schwefelsäure ausgeführt wird.

5. Material nach einem der Ansprüche 1 bis 4, **dadurch gekennzeichnet, daß** das Substrat eine Verbindung ist, ausgewählt aus der Gruppe bestehend aus Polypropylen, Polyvinylchlorid und Polyurethan.

6. Material nach Anspruch 1 , **dadurch gekennzeichnet, daß** das Substrat ein Polyamid/Polyether-Copolymer ist.

7. Material nach einem der Ansprüche 1 bis 6, **dadurch gekennzeichnet, daß** das Verknüpfungsmittel Glutaraldehyd ist.

8. Material nach Anspruch 2 , **dadurch gekennzeichnet, daß** das Copolymer, das Hydroxyethylmethacrylat (HEMA) und Methylmethacrylat (MMA) enthält, nicht weniger als 30% Hydroxyethylmethacrylat (HEMA) enthält.

9. Medizinisches Gerät, wobei mindestens ein Bereich davon, der mit dem Blut in Berührung kommt, aus dem Anti-Material nach einem der Ansprüche 1 bis 8 gebildet ist.

10. Hohlfaser, wobei mindestens ein Bereich davon, der mit dem Blut in Berührung kommt, aus dem Material nach einem der Ansprüche 1 bis 8 gebildet ist.

11. Oxygenierungsvorrichtung, umfassend eine poröse Membran mit einer Vielzahl an Mikroporen als eine Gasaustauschmembran, wobei eine Oberfläche der Membran, auf der das Blut zirkuliert, aus dem Material nach einem der Ansprüche 1 bis 8 gebildet ist.

12. Oxygenierungsvorrichtung nach Anspruch 11, wobei eine Vielzahl an feinen Teilchen mit einer Teilchengröße, die kleiner als der Porendurchmesser ist, in die Mikroporen der porösen Membran eingebracht ist.

13. Oxygenierungsvorrichtung nach Anspruch 12, wobei die feinen Teilchen Siliciumdioxidteilchen sind.

14. Verfahren zur Herstellung eines medizinischen Materials nach Anspruch 1, das die nachstehenden Schritte umfaßt:
(a) Behandlung eines Polymersubstrats mittels Ozonisierung unter Einführung von funktionellen Gruppen,
(b) Bindung einer Verbindung mit zwei oder mehreren primären Aminogruppen an die funktionellen Gruppen, und
(c) kovalentes Verbinden der Aminogruppen des oxidierten Substrats und der Aminogruppen des partiell desulfurierten Heparins mittels eines Verknüpfungsmittels.

15. Verfahren zur Herstellung eines medizinischen Materials nach Anspruch 2, das die nachstehenden Schritte umfaßt:
(a) Beschichten eines Substrats mit einem Copolymer aus HEMA und MMA, das aus getrennten Segmenten aus HEMA und MMA gefertigt ist, wobei das HEMA-Segment eine Verbindung mit Epoxygruppen enthält,
(b) Umsetzen einer Verbindung mit zwei oder mehreren primären Aminogruppen mit dem beschichteten Substrat, und
(c) kovalentes Verbinden der Aminogruppen des Substrats und der Aminogruppen des partiell desulfurierten Heparins mittels eines Verknüfungsmittels.

16. Verfahren nach einem der Ansprüche 14 oder 15, wobei das Verknüpfungsmittel eine Verbindung mit mindestens zwei Aldehydgruppen ist.

17. Verfahren nach Anspruch 16, wobei die Verbindung mit mindestens zwei Aldehydgruppen Glutaraldehyd ist.

18. Verfahren nach einem der Ansprüche 15-17, wobei die Verbindung, die die Epoxygruppen enthält, Glycidylmethacrylat ist.

19. Verfahren zur Herstellung einer Oxygenierungsvorrichtung nach Anspruch 11, wobei das Material nach dem Verfahren nach einem der Ansprüche 14-18 hergestellt wird.

**Revendications**

1. Matériau médical comprenant un support polymère et de l'héparine immobilisée sur celui-ci pour conférer au support des propriétés anti-thrombotiques, caractérisé en ce que le support a des groupes fonctionnels introduits par ozonation, au moyen desquels un composé ayant deux groupes amino primaires (A) ou plus est couplé et l'héparine a des groupes amino primaires (B) obtenus par désulfatation partielle de ses restes N-sulfates, lesdits groupes amino primaires (B) dudit dérivé d'héparine étant liés de manière covalente auxdits groupes amino primaires (A) avec au moins un agent de couplage ayant des groupes réagissant avec les groupes amino primaires (A) et (B).

2. Matériau médical comprenant un support et de l'héparine immobilisée sur celui-ci pour conférer au support des propriétés anti-thrombotiques, caractérisé en ce que le support est revêtu avec un copolymère de méthacrylate d'hydroxyéthyle (HEMA) et de méthacrylate de méthyle (MMA) formé de séquences séparées de HEMA et de MMA, ladite séquence de HEMA comprenant des groupes époxy avec lesquels un composé ayant deux groupes amino primaires (A) ou plus est mis à réagir et l'héparine a des groupes amino primaires (B) obtenus par désulfatation partielle de ses restes N-sulfates, lesdits groupes amino primaires (B) dudit dérivé d'héparine étant liés de manière covalente auxdits groupes amino primaires (A) avec au moins un agent de couplage ayant des groupes réagissant avec les groupes amino primaires (A) et (B).

3. Matériau selon l'une des revendications 1 ou 2, dans lequel ladite héparine désulfaté a entre 5 et 15 % de groupes amino primaires, par rapport à la somme des groupes amino primaires initiaux et des restes N-sulfates initiaux dans l'héparine native.

4. Matériau selon l'une quelconque des revendications 1 à 3, dans lequel ladite désulfatation partielle est effectuée dans l'acide sulfurique aqueux.

5. Matériau selon l'une quelconque des revendications 1 à 4, dans lequel ledit support est choisi parmi le polypropylène, le chlorure de polyvinyle et le polyuréthanne.

6. Matériau selon la revendication 1, dans lequel ledit support est un copolymère polyamide/polyéther.

7. Matériau selon l'une quelconque des revendications 1 à 6, dans lequel ledit agent de couplage est le glutaraldéhyde.

8. Matériau selon la revendication 2, dans lequel ledit copolymère contenant du méthacrylate d'hydroxyéthyle (HEMA) et du méthacrylate de méthyle (MMA) ne contient pas moins de 30 % de méthacrylate d'hydroxyéthyle (HEMA).

9. Dispositif médical dans lequel au moins une portion de celui-ci en contact avec le sang est formée du matériau selon l'une quelconque des revendications 1 à 8.

10. Fibre creuse dans laquelle au moins une portion de celle-ci en contact avec le sang est formée du matériau selon l'une quelconque des revendications 1 à 8.

11. Oxygénateur comprenant une membrane poreuse ayant de nombreux micropores comme membrane d'échange de gaz, dans lequel une surface de la membrane sur laquelle on fait circuler le sang est formée du matériau selon l'une quelconque des revendications 1 à 8.

**12.** Oxygénateur selon la revendication 11 dans lequel lesdits micropores de ladite membrane poreuse sont garnis avec de nombreuses fines particules ayant une dimension de particule inférieure au diamètre des pores.

**13.** Oxygénateur selon la revendication 12, dans lequel lesdites fines particules sont des particules de silice.

**14.** Procédé pour la production d'un matériau médical selon la revendication 1 qui comprend les étapes suivantes :

(a) on traite un support de polymère par ozonation pour y introduire des groupes fonctionnels ;

(b) on effectue le couplage d'un composé ayant deux groupes amino primaires ou plus avec lesdits groupes fonctionnels ; et

(c) on lie de manière convalente lesdits groupes fonctionnels du support oxydé et les groupes amino de l'héparine partiellement désulfatée au moyen d'un agent de couplage.

**15.** Procédé pour la production d'un matériau médical selon la revendication 2 qui comprend les étapes suivantes :

(a) on recouvre un support avec un copolymère de HEMA et de MMA constitué de séquences séparées de HEMA et de MMA, la séquence de HEMA contenant un composé ayant des groupes époxy ;

(b) on fait réagir un composé ayant deux groupes amino primaires ou plus avec ledit support revêtu ; et

(c) on lie de manière covalente les groupes amino du support et les groupes amino de l'héparine partiellement désulfatée au moyen d'un agent de couplage.

**16.** Procédé selon l'une quelconque des revendications 14 ou 15 dans lequel ledit agent de couplage est un composé ayant au moins deux groupes aldéhydes.

**17.** Procédé selon la revendication 16 dans lequel ledit composé ayant au moins deux groupes aldéhydes est le glutaraldéhyde.

**18.** Procédé selon l'une quelconque des revendications 15 à 17 dans lequel ledit composé contenant des groupes époxy est le méthacrylate de glycidyle.

**19.** Procédé pour produire un oxygénateur selon la revendication 11 dans lequel ledit matériau est préparé par le procédé selon l'une quelconque des revendications 14 à 18.

# F I G . 1

# F I G. 2

# F I G . 3

# F I G. 4